# EUROPÄISCHE PATENTSCHRIFT

(11) **EP 2 453 933 B1**
(45) Veröffentlichungstag und Bekanntmachung des Hinweises auf die Patenterteilung: **31.08.2016**
(21) Anmeldenummer: 10735194.2
(22) Anmeldetag: 03.07.2010
(51) Int. Cl.: A61L 15/24, A61L 15/44, A61L 15/58, A61L 15/34

(54) **WASSERDAMPFDURCHLÄSSIGES HAUTPFLASTER**
WATER-VAPOR PERMEABLE ADHESIVE BANDAGE
PANSEMENT CUTANÉ PERMÉABLE À LA VAPEUR D'EAU

(30) Priorität: 14.07.2009 DE 102009032866; 12.11.2009 DE 102009052943
(43) Veröffentlichungstag der Anmeldung: 23.05.2012
(73) Patentinhaber: LTS LOHMANN Therapie-Systeme AG, 56626 Andernach (DE)
(72) Erfinder: HORSTMANN, Michael, 56564 Neuwied (DE); HAUSEN, Christian, DE / 53562 St. Katharinen (DE); MOHR, Patrick, 53498 Bad Breisig (DE); LUDWIG, Karin, 56589 Datzeroth (DE); KLEUDGEN, Tobias, 56729 Ettringen (DE)
(74) Vertreter: Schweitzer, Klaus
(86) Internationale Anmeldenummer: PCT/EP2010/004029
(87) Internationale Veröffentlichungsnummer: WO 2011/006593

(56) Entgegenhaltungen:
- EP-A1- 0 157 960
- EP-A2- 0 730 874
- EP-A2- 0 739 626
- WO-A1-03/002684
- WO-A1-2005/051333
- WO-A2-2008/063623

## Beschreibung

Die vorliegende Erfindung betrifft Hautpflaster, das heißt Pflaster zum Bedecken eines Bereichs der Oberfläche eines Lebewesens, die an der Haut haften können, nach Patentanmeldung DE 10 2009 032 866.1. Die Erfindung betrifft insbesondere Hautpflaster mit einer hohen Wasserdampfdurchlässigkeit.

Üblicherweise umfassen Pflaster zur Behandlung oder Vorbeugung von Wunden oder Druckstellen eine Rückschicht und eine Klebschicht. Die Rückschicht kann okklusiv sein, das heißt wasserdampfundurchlässig, und dadurch einen Anstieg unerwünschter Feuchtigkeit unterhalb (= im Bereich der Applikationsstelle) des Hautpflasters verursachen, was zu Hautirritationen und verkürzter Tragzeit führt.

Es hat zahlreiche Versuche gegeben, Hautpflaster mit einer erhöhten Wasserdampfdurchlässigkeit zu entwickeln, die ein Verdunsten von auf der Oberfläche der Haut auftretender Feuchtigkeit durch das Hautpflaster ermöglichen. Dadurch kann eine unerwünschte Ansammlung von Feuchtigkeit und die damit einhergehende Ablösung des Hautpflasters oder Vermehrung von Bakterien unterhalb des Hautpflasters vermieden werden.

Das Problem für den Fachmann bei der Entwicklung von wasserdampfdurchlässigen Hautpflastern besteht darin, einen hautverträglichen Haftkleber bereitzustellen, der gleichzeitig eine hohe Klebkraft und eine hohe Wasserdampfdurchlässigkeit und/oder Wasseraufnahmekapazität aufweist.

Im Stand der Technik werden beispielsweise wasserhaltige Kleber offenbart, die nahezu vollständig auf wasserlöslichen Polymeren basieren. Allerdings wird die Klebkraft der wasserhaltigen Kleber als unzureichend empfunden. Außerdem lösen sich Hautpflaster mit einer Klebschicht auf Basis von wasserlöslichen Polymeren nach einigen Stunden Tragzeit ab, weil die Klebschicht austrocknet und dadurch ihre Klebkraft verliert.

Im Stand der Technik werden auch Hydrokolloid-Klebmassen beschrieben, um nicht-okklusive Hautpflaster herzustellen. Klassische Hydrokolloid-Klebmassen werden traditionell aus pflanzlichen Gummen unter Zusatz weiterer Naturprodukte erzeugt und in der Regel in dicker Schicht aufgebracht. Solche Massen werden beispielsweise als Randklebemassen für Kolostomie-Beutel verwendet.

Besonders geeignete Hydrokolloid-Klebmassen werden in US 3,339,546 beschrieben, insbesondere eine Klebmasse, umfassend Polyisobutylene und ein oder mehrere Hydrokolloide, die in wässrigen Flüssigkeiten quellen kann.

Weitere Beispiele für Hydrokolloid-Klebmassen sind aus US 4,231,369 und US 4,367,632 bekannt.

US 4,231,369 offenbart ein Siegelmaterial für Kolostomie-Beutel, umfassend mindestens ein vernetztes Elastomer als Basis für eine äußere, kontinuierliche Phase, in der mindestens ein Hydrokolloid dispergiert ist. Bei dem Elastomer kann es sich um ein Styrol-Olefin-Styrol-Blockcopolymer oder um ein Ethylen-Propylen-Block-Copolymer handeln. Zudem enthält die äußere Phase einen Klebrigmacher aus der Gruppe der Polymere und Copolymere des Dicyclopentadiens, alpha-Pinens oder beta-Pinens.

US 4,367,732 betrifft Hautpflaster, bestehend aus einem elastischen Film und einem schwach elastischen Klebstoff. Dieser Klebstoff umfasst:
(I) eine kontinuierliche Phase, umfassend
   (a) ein physikalisch vernetztes Elastomer in Form eines Styrol-Isopren-Styrol-Block-Copolymers oder Ethylen-Propylen-Block-Copolymers,
   (b) ein Kohlenwasserstoff-Harz in Form eines Polymers oder Copolymers des Cyclopentadiens, Dicyclopentadiens, alpha-Pinens und/oder beta-Pinens,
   (c) ein Antioxidationsmittel,
   (d) optional ein Öl-Streckmittel, bestehend aus einem oder mehreren Mineralölen, und
   (e) optional einen Weichmacher, der polar zudem Elastomer ist, zum Beispiel einem Ester eines Polyethylenglykols oder Polypropylenglykols, oder einem Ester einer di- oder polybasigen Carbonsäure mit einem vorzugsweise aliphatischen Alkohol, und
(II) eine in der kontinuierlichen Phase dispergierte Phase, umfassend ein oder mehrere wasserquellbare Hydrokolloide.

Der elastische Film, der die Rückschicht bildet, soll wasserundurchlässig sein, wobei ein elastischer, wasserundurchlässiger Polyurethan-Film bevorzugt wird.

Der Klebstoff kann bei dem Hautpflaster in Form einer relativ dünnen Schicht vorliegen, die eine Dicke von 0,25 bis 3 mm aufweist und vorzugsweise eine Dicke von etwa 1,1 mm hat .

In jüngerer Zeit wurden auch sehr dünne Hautpflaster mit und ohne Zusatz von wasserguellbaren Partikeln entwickelt. So offenbart die WO 2006/130461 A1 dünne, nicht-okklusive Hautpflaster zur Behandlung von virusbedingten Läsionen. Diese Hautpflaster umfassen eine Rückschicht und eine Klebschicht, die im Wesentlichen frei von Hydrokolloid-Partikeln ist. Die Hautpflaster sollen eine Dicke von nur 10 bis 1.500 µm aufweisen, wobei beschrieben wird, dass die Klebschicht zwischen 20 und 200 µm dick sein kann.

In der WO 2005/051333 A1 werden Hautpflaster beschrieben, die eine wasserdampfdurchlässige Rückschicht und eine hautfreundliche, Hydrokolloid-Partikel enthaltende Klebschicht aufweisen. Die Dicke der Klebschicht dieser Hautpflaster beträgt zumindest im Kantenbereich des Hautpflasters 20 bis 300 µm, die Wasserdampfdurchlässigkeit der Hautpflaster liegt bei 200 bis 1000 g/m² und die Feuchtigkeitsabsorption des Hautpflasters beträgt 40 bis 600 g/m²/6h.

In der WO 03/002684 A1 werden elastische Klebstoffe beschrieben, die 3 bis 30 Gew.-% eines Styrol-Isopren-Styrol-Block-Copolymeren und bis zu 25 Gew.-% eines Esterharzes des Kolophoniums, wie z.B. einen Ester des Kolophoniums mit Pentaerythrit oder Glycerin, umfassen.

Die aus dem Stand der Technik bekannten Pflaster weisen jedoch keine zufriedenstellende Klebkraft bei ausreichend hoher Wasserdampfdurchlässigkeit auf.

Die der vorliegenden Erfindung zugrunde liegende Aufgabe bestand daher darin, Hautpflaster bereitzustellen, die eine gute Klebkraft bei gleichzeitig hoher Wasserdampfdurchlässigkeit aufweisen.

Die Aufgabe wird überraschenderweise durch ein Hautpflaster gelöst, das eine Rückschicht aus einem wasserdampfdurchlässigen Material, vorzugsweise ein Film aus Polyurethan, und eine Klebschicht umfasst, die 10 Gew.-% einer dispersen Innenphase aus hydrophilen, wasserquellbaren Partikeln in einer Außenphase enthält, umfassend mindestens 10 Gew.-% eines Styrol-Block-Copolymers und mindestens 20 Gew.-% aus einem Esterharz des Kolophoniums.
Die Rückschicht des erfindungsgemäßen Hautpflasters ist wasserdampfdurchlässig. Grundsätzlich geeignete Materialien für eine Rückschicht sind Polyolefin-Folien, beispielsweise aus Polyethylen oder Polypropylen, Polyvinylchlorid-Folien, Polyetheramid-Folien, Polyamid-Folien, Polyester-Folien, Ethylen-Vinylacetat-Folien, Gewebe, Gewirke, Schäume und Polyurethan-Folien. Im Sinne der Erfindung wird der Begriff "Folien" gleichermaßen für Polymer-Folien wie für PolymerFilme verwendet.

Die Rückschicht kann monolithisch sein, sofern sie die erforderliche Wasserdampfdurchlässigkeit aufweist. Die Rückschicht kann jedoch auch mikroporös oder löchrig sein, um eine ansonsten wasserdampfundurchlässige Polymer-Folie wasserdampfdurchlässig zu machen.

Vorzugsweise ist die Rückschicht wasserundurchlässig, jedoch wasserdampfdurchlässig.

Bei dem bevorzugten Material für die Rückschicht handelt es sich um eine reibungsarme, flexible Polymerfolie. Besonders bevorzugte Materialien für die Rückschicht sind wasserdampfdurchlässige Polyurethan-Folien.
Die Rückschicht weist eine geeignete Dicke für die vorgesehene Verwendung auf. Die Verwendung dünnerer Rückschichten führt zu Hautpflastern mit besserer Dehnbarkeit und Anpassungsfähigkeit an die Körperoberfläche.

Vorzugsweise weist die Rückschicht eine Dicke von weniger als 30 *µ*m auf, besonders bevorzugt von weniger als 20 *µ*m und ganz besonders bevorzugt von weniger als 18 *µ*m. Die Rückschicht kann sogar eine Dicke von weniger als 15 *µ*m aufweisen, beispielsweise eine Dicke von 12 *µ*m.

Die Wasserdampfdurchlässigkeit der Rückschicht beträgt mindestens 200 g/24 Stunden, vorzugsweise mindestens 800 g/24 Stunden. Die Wasserdampfdurchlässigkeit beträgt bis zu 1.300 g/24 Stunden, vorzugsweise bis zu 6.000 g/24 Stunden.

Die Klebschicht des erfindungsgemäßen Hautpflasters besteht aus einem Klebstoff, der eine kontinuierliche äußere Phase und eine in dieser äußeren Phase dispergierte innere Phase aufweist. Die kontinuierliche äußere Phase umfasst mindestens 10 Gew.-% eines Styrol-Block-Copolymers und mindestens 20 Gew.-% eines Esterharzes des Kolophoniums. Die Angabe der Gewichtsprozente bezieht sich auf die Masse des Klebstoffs. Die innere Phase besteht aus hydrophilen, wasserquellbaren Partikeln und macht mindestens 10 Gew.-% der Klebstoffmasse aus.
Der Klebstoff für die Klebschicht der erfindungsgemäßen Hautpflaster enthält ein Styrol-Block-Copolymer. Bei dem Styrol-Block-Copolymer handelt es sich vorzugsweise um ein Elastomer aus der Gruppe der Styrol-Olefin-Styrol-BlockCopolymere. Der Olefin-Block kann auf Isopren, Butadien, anderen kurzkettigen Alkadienen oder Alkanen, wie beispielsweise Mischungen aus Ethylen und Butylen, oder Polyisobutylen, sowie aus Kombinationen dieser Verbindungen basieren. Für die äußere Phase besonders bevorzugte Styrol-Block-Copolymere sind Styrol-Isopren-Styrol-BlockCopolymere.

Der Klebstoff für die Klebschicht der erfindungsgemäßen Hautpflaster enthält mindestens ein Esterharz des Kolophoniums.

Kolophonium ist ein natürliches Harz, das aus dem Balsam von Koniferen gewonnen wird. Es handelt sich um den nicht destillierbaren, festen Rückstand des Koniferenbalsams. Das Destillat wird als Terpentinöl bezeichnet und enthält als Hauptbestandteile alpha-Pinen und beta-Pinen. Kolophonium besteht aus einem Gemisch von Harzsäuren und Terpenen. Hauptbestandteil des Kolophoniums sind leicht zu oxidierende Harzsäuren, wie Abietinsäure und Pimarsäure. Indem die Harzsäuren des Kolophoniums mit Polyolen wie Pentaerythrit, Glycerol oder Glykole umgesetzt werden, erhält man die so genannten Kolophoniumester, die auch als Esterharze des Kolophoniums bezeichnet werden.

Daher umfasst der Begriff "Esterharze des Kolophoniums" im Sinne der vorliegenden Erfindung auch Ester der Harzsäuren, insbesondere Ester der Abietinsäure und/oder der Pimarsäure, zu denen ausdrücklich auch die Ester mit Pentaerythrit, Glycerol oder Glykolen zählen.

Die Herstellung von Kolophoniumestern findet in der Regel durch Chargenverfahren in geschmolzenem Zustand bei sehr hohen Temperaturen (250 bis 300 °C) statt.

Vorzugsweise handelt es sich bei den Esterharzen des Kolophoniums für die erfindungsgemäßen Hautpflaster um Ester des Glycerins, Ester des Pentaerythrits oder Mischungen dieser Ester.

Der Klebstoff für die Klebschicht der erfindungsgemäßen Hautpflaster enthält hydrophile, wasserquellbare Partikel. Vorzugsweise handelt es sich bei den hydrophilen, wasserquellbaren Partikeln um Hydrokolloid-Partikel.

Unter "Hydrokolloiden" versteht man eine große Gruppe von Polysacchariden und Proteinen, die in Wasser als Kolloide in Lösung gehen und ein hohes Vermögen zur Gelbildung zeigen. Fast alle Hydrokolloide sind natürlichen Ursprungs. Entweder handelt es sich um natürlich vorkommende hydrophile Polymere oder um deren chemisch modifizierte Varianten. Zu den Hydrokolloiden zählen beispielsweise Stärke, Cellulosen wie Carboxymethylcellulose, Chitosan, Pektine, Gummi Arabicum, Guarkernmehl, Johannisbrotkernmehl, Agar, Carrageen, Alginate, Gelatine, Caseinate, Dextrine und Xanthan.

Für die hydrophilen, wasserquellbaren Partikel sind auch synthetische Polymer aus den gleichen oder unterschiedlichen Monomeren geeignet. Geeignete hydrophile, wasserquellbare Partikel können zum Beispiel aus Polyacrylsäure, Polyvinylalkohol, Polyvinylacetat, Polyhydroxyalkylacrylaten, Polyhydroxyalkylmethacrylaten, Polyacrylamiden, Polystyrolsulfonaten, Polyvinylpyrrolidon, Polyglykolen, Copolymeren, Pfropf-Copolymeren und Mischungen der genannten Polymere bestehen.

Die hydrophilen, wasserquellbaren Partikel weisen eine durchschnittliche Größe von weniger als 125 *µ*m auf, vorzugsweise sind sie kleiner als 100 *µ*m, besonders bevorzugt kleiner als 75 *µ*m und ganz besonders bevorzugt kleiner als 50 *µ*m.

Durch den Gehalt an hydrophilen, wasserquellbaren Partikeln in der Klebschicht können mit den erfindungsgemäßen Hautpflastern gute Bedingungen für eine feuchte Wundheilung erreicht werden.

Die Klebschicht des erfindungsgemäßen Hautpflasters kann relativ dick sein, beispielsweise bis zu 1 mm, vorzugsweise bis zu 3 mm und besonders bevorzugt bis zu 5 mm.

Gemäß bevorzugter Ausführungsformen handelt es sich bei der Klebschicht der erfindungemäßen Hautpflaster jedoch um eine vergleichsweise dünne Schicht, die nicht dicker als 300 *µ*m, vorzugsweise nicht dicker als 200 *µ*m, besonders bevorzugt nicht dicker als 150 *µ*m und ganz besonders bevorzugt nicht dicker als 100 *µ*m ist. Als Untergrenze für die Dicke der Klebschicht können 25 *µ*m, vorzugsweise 30 *µ*m angegeben werden. Dünne Klebschichten werden bevorzugt, weil auch das resultierende Hautpflaster dünn und dadurch flexibler ist.

Die Dicke der Klebschicht des erfindungsgemäßen Hautpflasters kann über gesamte Oberfläche des Hautpflasters im Wesentlichen konstant sein. In bevorzugten Ausführungsformen ist die Klebschicht in der Mitte des Hautpflasters jedoch dicker als am Randbereich. Eine sich zur Kante des Hautpflasters hin verjüngende Klebschicht vermeidet ein Sich-Abrollen des Hautpflasters von der Haut und verlängert somit die mögliche Tragzeit. Bei dünnen Klebschichten kann jedoch auch auf eine Verjüngung am Randbereich verzichtet werden, ohne dass die mögliche Tragzeit wesentlich beeinträchtigt wird.

Die erfindungsgemäßen Hautpflaster sind wasserdampfdurchlässig. Die Wasserdampfdurchlässigkeit der erfindungsgemäßen Hautpflaster liegt bei mindestens 50 g/m², vorzugsweise bei mindestens 80 g/m², besonders bevorzugt bei mindestens 100 g/m², in einem Zeitraum von 24 Stunden. Die Wasserdampfdurchlässigkeit der erfindungsgemäßen Hautpflaster liegt unterhalb von 1000 g/m², bei besonderen Ausführungsformen unterhalb von 400 g/m², vorzugsweise unterhalb von 250 g/m², besonders bevorzugt unterhalb von 150 g/m², bezogen auf einen Zeitraum von 24 Stunden.

Die erfindungsgemäßen Hautpflaster sind aufgrund der hydrophilen, wasserquellbaren Partikel in der Klebschicht in der Lage, eine bestimmte Menge Feuchtigkeit aufzunehmen.

Die Menge an Feuchtigkeit, die absorbiert werden kann, ist unter anderem abhängig von der Dicke der Klebschicht, dem Material der hydrophilen, wasserquellbaren Partikel und ihrem Gehalt in der Klebschicht.

In bevorzugten Ausführungsformen weist das erfindungsgemäße Hautpflaster eine Absorptionskapazität von mindestens 40 g/m²/6h, vorzugsweise von mindestens 50 g/m²/6h, besonders bevorzugt von mindestens 60 g/m²/6h und ganz besonders bevorzugt von mindestens 70 g/m²/6h. Die Absorptionskapazität beträgt bis zu 250 g/m²/6h, vorzugsweise bis zu 300 g/m²/6h, besonders bevorzugt bis zu 400 g/m²/6h und ganz besonders bevorzugt bis zu 600 g/m²/6h. Die Absorptionskapazität wird bestimmt, indem ein Hautpflaster vollständig in physiologische Kochsalz-Lösung (0,9 M NaCl in Wasser) bei einer Temperatur von 37 °C für eine Dauer von 6 Stunden eingetaucht und anschließend die aufgenommene Menge Wasser bestimmt wird.

Das erfindungsgemäße Hautpflaster kann eine Feuchtigkeit oder Wundexsudat absorbierende Auflage aufweisen. Die absorbierende Auflage befindet sich auf der Fläche der Klebschicht, die der Rückschicht gegenüber liegt. Die absorbierende Auflage kann aus jedem beliebigen geeigneten Material, beispielsweise Gaze, Alginate, Kollagene, Schäume, Superabsorbern oder dergleichen bestehen. Derartige Auflagen und die für sie geeigneten Materialien sind dem Fachmann bekannt.

Die erfindungsgemäßen Hautpflaster eigenen sich zur Abdeckung von Schnitt- oder Schürfwunden sowie von Narben, anderen Hautläsionen und dergleichen.

In einer besonderen Ausführungsform enthalten die erfindungsgemäßen Hautpflaster einen antiviralen Wirkstoff oder eine Mischung antiviraler Wirkstoffe in der Klebschicht.

Der antivirale Wirkstoff, mindestens einer der antiviralen Wirkstoffe oder die antiviralen Wirkstoffe ist/sind vorzugsweise aus der Gruppe von Wirkstoffen ausgewählt, die Aciclovir, Valaciclovir, Penciclovir, Famciclovir, Brivudin, Adeninarabinosid und Phosphonameisensäure, auch unter dem Freinamen Foscarnet bekannt, umfasst.

Der antivirale Wirkstoff, mindestens einer der antiviralen Wirkstoffe oder die antiviralen Wirkstoffe können auch aus der Gruppe von Wirkstoffen ausgewählt sein, die Kieselsäure, n-Docosanol und Idoxuridin umfasst. Diese Wirkstoffe gelten zwar nicht primär als antiviral, weisen aber dennoch eine gute antivirale Wirksamkeit auf.

Der Gehalt an antiviralem Wirkstoff beziehungsweise an antiviralen Wirkstoffen in der Klebschicht beträgt mindestens 0,1 Gew.-%, vorzugsweise mindestens 1,0 Gew.-%, bezogen auf die Masse der wirkstoffhaltigen Klebschicht. Die wirkstoffhaltige Klebschicht kann bis zu 20 Gew.-% Wirkstoff enthalten, enthält aber vorzugsweise nicht mehr als 10 Gew.-%, jeweils bezogen auf die Masse der wirkstoffhaltigen Klebschicht. Dem Fachmann ist bekannt, dass die zu verwendende Wirkstoffmenge von dem zu verwendenden Wirkstoff oder der zu verwendenden Wirkstoffmischung abhängt.

Die Ausführungsformen der erfindungsgemäßen Hautpflaster, die mindestens einen antiviralen Wirkstoff enthalten sind zur Abdeckung von Hautläsionen geeignet, die durch eine Infektion mit Viren hervorgerufen werden, beispielsweise die so genannten "Herpes-Bläschen" bei einer Infektion mit *Herpes simples labialis.*

Die erfindungsgemäßen Hautpflaster weisen eine ablösbare Schutzschicht oder Schutzfolie ("release liner") auf, welche die Klebschicht auf ihrer der Rückschicht gegenüberliegenden Fläche abdeckt. Die Klebfläche kann vollständig von einer einteiligen Schutzschicht abgedeckt sein oder mehrere Schutzschichten können die Klebefläche teilweise abdecken, wobei bevorzugt ist, dass die gesamte Klebefläche abgedeckt ist. Die Schutzschicht(en) oder Schutzfolie(n) ist/sind vor oder bei dem Anbringen des Hautpflasters auf der Haut zu entfernen.

Als Material für die Schutzschicht eignen sich beispielsweise silikonisiertes Papier oder Kunststofffolien, beispielsweise aus Polyethylenterephthalat, Polyester, Polyethylen, die gegebenenfalls mit einer abhäsiven Beschichtung versehen sind. Materialien für eine Schutzschicht zum Abdecken einer Klebschicht von Hautpflastern sind dem Fachmann bekannt.

Es ist nicht notwendig, dass die Schutzschicht die gleiche Größe und/oder Form wie das Hautpflaster hat. Es ist beispielsweise möglich, mehrere Hautpflaster auf einer einzigen Schutzschicht oder mehreren gemeinsamen Schutzschichten aufzubringen.

Insbesondere eine einteilige Schutzschicht kann auch als Teil der Primärverpackung für das erfindungsgemäße Hautpflaster ausgebildet sein.

Die erfindungsgemäßen Hautpflaster lassen sich unter Nutzung bekannter Verfahren zur Herstellung von Hautpflastern herstellen.

Die erfindungsgemäßen Hautpflaster eignen sich hervorragend für eine Verwendung als Abdeckung von Schnitt- oder Schürfwunden, Narben, Hautläsionen und dergleichen, wobei sie eine gute Wasserdampfdurchlässigkeit und gleichzeitig eine hohe Klebkraft aufweisen. Die erfindungsgemäßen Hautpflaster zeichnen sich somit durch eine lange Tragzeit aus und mit ihnen können auch optimale Bedingungen für eine feuchte Wundheilung geschaffen werden, ohne dass es zu einer unerwünschten Ansammlung von Feuchtigkeit unterhalb des Hautpflasters kommt.
Die Erfindung wird durch folgende Ausführungsbeispiele veranschaulicht:

### Beispiel 1:

Zu 288,01 g Spezialbenzin 80/110 werden unter Rühren 289,56 g Foral 85E (ein Glycolester des dehydrierten Kolophoniums) gegeben und homogenisiert. Dann erfolgt Zugabe von 99,23 g Kraton D 1161 NU (einem linearen Block-Copolymer auf Basis von Styrol und Isopren). Die Mischung wird homogenisiert. Schließlich werden 43,2 g Na-Carboxymethylcellulose (CRT 60000 SPA 07) zugegeben und bis zur vollständigen Homogenität gerührt.

Die Mischung wird in einer Strichstärke von 200 *µ*m auf eine Polyurethan-Folie auf Stützpapier (Typ U 50, 55 g/m² von Smith & Nephew) ausgestrichen. Die schichtförmige Masse wird im Trockenschrank 40 min bei Raumtemperatur und danach 30 min bei 60°C getrocknet. Es ergibt sich ein Flächengewicht von 100 g/m². Man deckt ab mit einem Release Liner (Marke: Silphan 100 *µ*m tsp AB1, 140 g/m²).

### Beispiel 2:

Zu 72,0 g Spezialbenzin 80/110 werden unter Rühren 72,4 g Foral 85E und in drei Portionen 24,8 g Kraton D1161 zugegeben. Der Masse werden anschließend 10,8 g Natriumcarboxymethylcellulose zugefügt und unter Rühren homogenisiert. Die blasenfreie Masse wird mit einer Strichstärke von 200 *µ*m auf eine silikonisierte PE-Folie aufgetragen und im Trockenschrank bei steigender Temperatur (1°/min) von Raumtemperatur auf 60°C gebracht.

Das resultierende Laminat mit einem Flächengewicht von 98 g/m² wird mit einer PU-Folie 50 *µ*m abgedeckt. Aus dem vorliegenden Laminat werden runde Pflaster mit einer Fläche von 1,77 cm² ausgestanzt.

## Patentansprüche

1. Hautpflaster, umfassend eine wasserdampfdurchlässige Rückschicht mit einer Wasserdampfdurchlässigkeit von mindestens 200 g/m²/24h und eine Klebschicht, die mindestens 10 Gew.-% einer dispersen Innenphase aus hydrophilen, wasserquellbaren Partikeln in einer Außenphase, umfassend mindestens 10 Gew.-% eines Styrol-Block-Copolymers und mindestens 20 Gew.-% aus einem Esterharz des Kolophoniums, umfasst, wobei die Wasserdampfdurchlässigkeit des Hautpflasters unterhalb von 1000 g/m² liegt, bezogen auf einen Zeitraums von 24 h.

2. Hautpflaster nach Anspruch 1, **dadurch gekennzeichnet, dass** das Material für die Rückschicht aus der Gruppe ausgewählt ist, die Polyethylen-Folien, Polypropylenfolien, Polyvinylchlorid-Folien, Polyetheramid-Folien, Polyamid-Folien, Polyester-Folien, Ethylen-Vinylacetat-Folien, Gewebe, Gewirke, Schäume und Polyurethan-Folien umfasst.

3. Hautpflaster nach Anspruch 1 oder 2, **dadurch gekennzeichnet, dass** die Rückschicht mikroporös oder löchrig ist.

4. Hautpflaster nach einem der vorhergehenden Ansprüche, **dadurch gekennzeichnet, dass** das Styrol-Block-Copolymer aus der Gruppe ausgewählt ist, die aus Styrol-Olefin-Styrol-Block-Copolymeren besteht.

5. Hautpflaster nach Anspruch 4, **dadurch gekennzeichnet, dass** der Olefin-Block auf einer oder mehreren Verbindungen basiert, die aus der Gruppe ausgewählt ist/sind, die Isopren, Butadien, Ethylen, Butylen, Polyisobutylen umfasst.

6. Hautpflaster nach einem der vorhergehenden Ansprüche, **dadurch gekennzeichnet, dass** das Styrol-Block-Copolymer ein Styrol-Isopren-Styrol-Block-Copolymer ist.

7. Hautpflaster nach einem der vorhergehenden Ansprüche, **dadurch gekennzeichnet, dass** das Esterharz des Kolophoniums aus der Gruppe ausgewählt ist, die Ester des Kolophoniums mit Glycerol, Ester des Kolophoniums mit Pentaerythrit und Ester des Kolophoniums mit Glykolen umfasst und/oder dass es sich bei dem Esterharz des Kolophoniums um einen Ester einer oder mehrerer Harzsäuren handelt, insbesondere um Ester der Abietinsäure und/oder Ester der Pimarsäure.

8. Hautpflaster nach einem der vorhergehenden Ansprüche, **dadurch gekennzeichnet, dass** es sich bei den hydrophilen, wasserquellbaren Partikeln um Hydrokolloide handelt, wobei bevorzugt die hydrophilen, wasserquellbaren Partikel aus einem Material bestehen, das aus der Gruppe von Verbindungen ausgewählt ist, die aus Stärkemehl, Cellulosen wie Carboxymethylcellulose, Chitosan, Pektin, Gummi Arabicum, Guarkernmehl, Johannisbrotkernmehl, Agar, Carrageen, Alginaten, Gelatine, Caseinate, Dextrine, Xanthan, Polyacrylsäuren,Polyvinylalkoholen, Polyvinylacetaten, Polyhydroxyalkylacrylaten, Polyhydroxyalkylmethacrylaten, Polyacrylamiden, Polystyrolsulfonaten, Polyvinylpyrrolidonen, Polyglykolen, Copolymeren, Pfropf-Copolymeren und Mischungen der genannten Polymere besteht.

9. Hautpflaster nach einem der vorhergehenden Ansprüche, **dadurch gekennzeichnet, dass** die Klebschicht nicht dicker als 300 µm, vorzugsweise nicht dicker als 200 µm, besonders bevorzugt nicht dicker als 150 µm und ganz besonders bevorzugt nicht dicker als 100 µm ist.

10. Hautpflaster nach einem der vorhergehenden Ansprüche, **dadurch gekennzeichnet, dass** die Wasserdampfdurchlässigkeit in einem Zeitraum von 24 Stunden mindestens 50 g/m², vorzugsweise mindestens 80 g/m², besonders bevorzugt mindestens 100 g/m² beträgt.

11. Hautpflaster nach einem der vorhergehenden Ansprüche, **dadurch gekennzeichnet, dass** die Absorptionskapazität des Hautpflasters mindestens 40 g/m²/6h, vorzugsweise mindestens 50 g/m²/6h, besonders bevorzugt mindestens 60 g/m²/6h und ganz besonders bevorzugt mindestens 70 g/m²/6h beträgt.

12. Hautpflaster nach einem der vorhergehenden Ansprüche, **dadurch gekennzeichnet, dass** es zusätzlich eine absorbierende Auflage aufweist.

13. Hautpflaster nach einem der vorhergehenden Ansprüche, **dadurch gekennzeichnet, dass** es zusätzlich eine ein- oder mehrteilige, ablösbare Schutzschicht oder Schutzfolie aufweist, die die Klebschicht auf ihrer der Rückschicht gegenüberliegenden Fläche ganz oder teilweise abdeckt.

14. Hautpflaster nach einem der vorhergehenden Ansprüche, **dadurch gekennzeichnet, dass** es mindestens einen antiviralen Wirkstoff enthält, wobei bevorzugt der antivirale Wirkstoff aus der Gruppe ausgewählt ist, die Aciclovir, Valaciclovir, Penciclovir, Famciclovir, Brivudin, Adeninarabinosid, Phosphonameisensäure, Kieselsäure, n-Docosanol und Idoxuridin umfasst.

15. Hautpflaster nach Anspruch 14, **dadurch gekennzeichnet, dass** der Gehalt an Wirkstoff(en) 0,1 bis 20 Gew.-% beträgt, vorzugsweise 1,0 bis 10 Gew.-%, jeweils bezogen auf die Masse der Klebschicht.

## Claims

1. A skin plaster comprising a water-vapour-permeable back layer having a water vapour permeability of at least 200 g/m²/24 h and an adhesive layer which comprises at least 10 % by weight of a disperse inner phase of hydrophilic, water-swellable particles in an outer phase comprising at least 10 % by weight of a styrene block copolymer and at least 20 % by weight of an ester resin of colophony, wherein the water vapour permeability of the skin plaster is below 1000 g/m², based on a period of 24 h.

2. The skin plaster according to claim 1, **characterised in that** the material for the back layer is selected from the group which comprises polyethylene films, polypropylene films, polyvinyl chloride films, polyether amide films, polyamide films, polyester films, ethylene vinyl acetate films, woven fabrics, knitted fabrics, foams and polyurethane films.

3. The skin plaster according to claim 1 or 2, **characterised in that** the back layer is microporous or perforated.

4. The skin plaster according to any one of the preceding claims, **characterised in that** the styrene block copolymer is selected from the group which consists of styrene-olefin-styrene block copolymers.

5. The skin plaster according to claim 4, **characterised in that** the olefin block is based on one or more compounds which is/are selected from the group which comprises isoprene, butadiene, ethylene, butylene, polyisobutylene.

6. The skin plaster according to any one of the preceding claims, **characterised in that** the styrene block copolymer is a styrene-isoprene-styrene block copolymer.

7. The skin plaster according to any one of the preceding claims, **characterised in that** the ester resin of colophony is selected from the group which comprises esters of colophony with glycerol, esters of colophony with pentaerythritol and esters of colophony with glycols, and/or **in that** the ester resin of colophony is an ester of one or more resin acids, in particular an ester of abietic acid and/or an ester of pimaric acid.

8. The skin plaster according to any one of the preceding claims, **characterised in that** the hydrophilic, water-swellable particles are hydrocolloids, wherein the hydrophilic, water-swellable particles consist of a material which is selected from the group of compounds which consists of starch flour, celluloses such as carboxymethyl cellulose, chitosan, pectin, gum arabic, guar seed flour, carob seed flour, agar, carrageenan, alginates, gelatin, caseinates, dextrins, xanthan, polyacrylic acids, polyvinyl alcohols, polyvinyl acetates, polyhydroxyalkyl acrylates, polyhydroxyalkyl methacrylates, polyacrylamides, polystyrenesulfonates, polyvinylpyrrolidones, polyglycols, copolymers, graft copolymers and mixtures of said polymers.

9. The skin plaster according to any one of the preceding claims, **characterised in that** the adhesive layer is not thicker than 300 µm, preferably not thicker than 200 µm, particularly preferably not thicker than 150 µm, and most preferably not thicker than 100 µm.

10. The skin plaster according to any one of the preceding claims, **characterised in that** water vapour permeability in a period of 24 hours is at least 50 g/m², preferably at least 80 g/m², particularly preferably at least 100 g/m².

11. The skin plaster according to any one of the preceding claims, **characterised in that** the absorption capacity of the skin plaster is at least 40 g/m²/6h, preferably at least 50 g/m²/6 h, particularly preferably at least 60 g/m²/6 h, and most preferably at least 70 g/m²/6 h.

12. The skin plaster according to any one of the preceding claims, **characterised in that** it additionally has an absorbent covering.

13. The skin plaster according to any one of the preceding claims, **characterised in that** it additionally has a single-part or multi-part, detachable protective layer or protective film which completely or partly covers the adhesive layer on its surface facing the back layer.

14. The skin plaster according to any one of the preceding claims, **characterised in that** it contains at least one antiviral active ingredient, wherein the antiviral active ingredient is preferably selected from the group which comprises aciclovir, valacyclovir, penciclovir, famciclovir, brivudine, adenine arabinoside, phosphonoformic acid, silicic acid, n-docosanol and idoxuridine.

15. The skin plaster according to claim 14, **characterised in that** the content of active ingredient(s) is 0.1 to 20 % by weight, preferably 1.0 to 10 % by weight, in each case based on the mass of the adhesive layer.

## Revendications

1. Pansement cutané comprenant une couche arrière perméable à la vapeur d'eau avec une perméabilité à la vapeur d'eau d'au moins 200 g/m²/24h et une couche adhésive, qui comprend au moins 10 % en poids d'une phase interne constituée de particules hydrophiles, pouvant gonfler dans l'eau, dispersée dans une phase externe, comprenant au moins 10 % en poids d'un copolymère séquencé de styrène et au moins 20 % en poids d'une résine ester de colophane, où la perméabilité à la vapeur d'eau du pansement cutané se situe en dessous de 1000 g/m² par rapport à une durée de 24 heures.

2. Pansement cutané selon la revendication 1, **caractérisé en ce que** le matériau pour la couche arrière est choisi dans le groupe qui comprend des films de polyéthylène, des films de polypropylène, des films de chlorure de polyvinyle, des films de polyéther amide, des films de polyamide, des films de polyester, des films d'éthylène vinylacétate, des tissus, des tricots, des mousses et des films de polyuréthane.

3. Pansement cutané selon les revendications 1 ou 2, **caractérisé en ce que** la couche arrière est microporeuse ou perforée.

4. Pansement cutané selon l'une des revendications précédentes, **caractérisé en ce que** le copolymère séquencé de styrène est choisi dans le groupe constitué des copolymères séquencés styrène-oléfine-styrène.

5. Pansement cutané selon la revendication 4, **caractérisé en ce que** la séquence oléfine est basée sur un ou plusieurs composés qui est/sont choisi/s dans le groupe qui comprend l'isoprène, le butadiène, l'éthylène, le butylène, le polyisobutylène.

6. Pansement cutané selon l'une des revendications précédentes, **caractérisé en ce que** le copolymère séquencé de styrène est un copolymère séquencé styrène-isoprène-styrène.

7. Pansement cutané selon l'une des revendications précédentes, **caractérisé en ce que** la résine ester de colophane est choisie dans le groupe qui comprend des esters du colophane avec le glycérol, des esters de colophane avec du pentaérythritol et des esters de colophane avec des glycols, et/ou qu'il s'agit d'un ester d'un ou de plusieurs acides résiniques dans le cas de la résine ester de colophane, notamment d'esters de l'acide abiétique et/ou d'esters de l'acide pimarique.

8. Pansement cutané selon l'une des revendications précédentes, **caractérisé en ce qu'**il s'agit d'hydrocolloïdes dans le cas des particules hydrophiles, pouvant gonfler dans l'eau, où de préférence les particules hydrophiles, pouvant gonfler dans l'eau sont constituées d'un matériau qui est choisi dans le groupes de composés qui est constitué de fécules, de celluloses comme la carboxyméthylcellulose, de chitosan, de pectine, de gomme arabique, de gomme de guar, de gomme de caroubier, d'agar-agar, de carraghénane, d'alginates, de gélatines, de caséinate, de dextrine, de xanthane, d'acides polyacryliques, d'alcools polyvinyliques, d'acétates de polyvinyle, d'acrylates de polyhydroxyalkyles, de méthacrylates de polyhydroxyalkyles, de polyacrylamides, de polystyrène sulfonates, de polyvinylpyrrolidones, de polyglycols, de copolymères, de copolymères ramifiés et de mélanges des polymères cités.

9. Pansement cutané selon l'une des revendications précédentes, **caractérisé en ce que** la couche adhésive n'est pas plus épaisse que 300 µm, de préférence pas plus épaisse que 200 µm, de manière particulièrement préférée pas plus épaisse que 150 µm, et idéalement pas plus épaisse que 100 µm.

10. Pansement cutané selon l'une des revendications précédentes, **caractérisé en ce que** la perméabilité à la vapeur d'eau pendant une durée de 24 heures s'élève à au moins 50 g/m², de préférence, à au moins 80 g/m², de manière particulièrement préférée à au moins 100 g/m².

11. Pansement cutané selon l'une des revendications précédentes, **caractérisé en ce que** la capacité d'absorption du pansement cutané s'élève à au moins 40 g/m²/6h, de préférence à au moins 50 g/m²/6h, de manière particulièrement préférée à au moins 60 g/m²/6h, et idéalement à au moins 70 g/m²/6h.

12. Pansement cutané selon l'une des revendications précédentes, **caractérisé en ce qu'**il présente en outre une couche absorbante.

13. Pansement cutané selon l'une des revendications précédentes, **caractérisé en ce qu'**il présente en outre une couche de protection amovible en une ou plusieurs parties ou un film de protection, qui recouvre totalement ou partiellement la surface située en face de sa couche arrière.

14. Pansement cutané selon l'une des revendications précédentes, **caractérisé en ce qu'**il contient au moins une matière active antivirale, où de préférence la matière active antivirale est choisie dans le groupe qui comprend l'aciclovir, le valaciclovir, le penciclovir, le famciclovir, la brivudine, l'adénine arabinoside, l'acide phosphonoformique, l'acide silicique, le n-docosanol et l'idoxuridine.

15. Pansement cutané selon la revendication 14, **caractérisé en ce que** la teneur en matière(s) active(s) s'élève de 0,1 à 20 % en poids, de préférence de 1,0 à 10 % en poids, respectivement par rapport à la masse de la couche adhésive.
